(19) Europäisches Patentamt
European Patent Office
Office européen des brevets

(11) **EP 1 434 572 B1**

(12) **FASCICULE DE BREVET EUROPEEN**

(45) Date de publication et mention
de la délivrance du brevet:
**13.12.2017 Bulletin 2017/50**

(51) Int Cl.:
***A61K 9/50*** *(2006.01)*

(21) Numéro de dépôt: **02800645.0**

(22) Date de dépôt: **09.10.2002**

(86) Numéro de dépôt international:
**PCT/FR2002/003443**

(87) Numéro de publication internationale:
**WO 2003/030878 (17.04.2003 Gazette 2003/16)**

(54) **FORME GALENIQUE ORALE MICROPARTICULAIRE POUR LA LIBERATION RETARDEE ET CONTROLEE DE PRINCIPES ACTIFS PHARMACEUTIQUES**

ORALE MIKROPARTIKULÄRE DOSIERUNGSFORM FÜR DIE VERZÖGERTE UND KONTROLLIERTE FREISETZUNG VON PHARMAZEUTISCHEN WIRKSTOFFEN

MICROPARTICULATE ORAL DOSAGE FORM FOR THE DELAYED AND CONTROLLED RELEASE OF PHARMACEUTICAL ACTIVE INGREDIENTS

(84) Etats contractants désignés:
**AT BE BG CH CY CZ DE DK EE ES FI FR GB GR IE IT LI LU MC NL PT SE SK TR**

(30) Priorité: **09.10.2001 FR 0112999**

(43) Date de publication de la demande:
**07.07.2004 Bulletin 2004/28**

(73) Titulaire: **Flamel Ireland Limited Dublin 15 (IE)**

(72) Inventeurs:
 • **LEGRAND, Valérie**
   **91330 Yerres (FR)**
 • **CASTAN, Catherine**
   **69510 Soucieu en Jarrest (FR)**
 • **MEYRUEIX, Rémi**
   **F-69009 Lyon (FR)**
 • **SOULA, Gérard**
   **F-69330 Meyzieu (FR)**

(74) Mandataire: **Nony**
   **11 rue Saint-Georges**
   **75009 Paris (FR)**

(56) Documents cités:
   **EP-A- 0 383 967    EP-A- 1 101 490**
   **WO-A-00/50015**

**Description**

Le domaine de la présente invention est celui des systèmes microparticulaires à libération retardée et contrôlée de principe(s) actif(s) PA, destinés à une administration par voie orale.

Les PA envisagés dans la présente invention sont ceux qui ont une absorption essentiellement limitée aux parties hautes du tractus gastro-intestinal, situées en amont du colon (de la jonction iléo-coecale), et qui représentent une grande majorité des principes actifs pharmaceutiques.

Plus précisément, l'invention se rapporte à une forme galénique microparticulaire à libération retardée et contrôlée pour laquelle la phase de libération contrôlée est déclenchée de façon certaine grâce à un double mécanisme : libération "temps dépendant" déclenchée au bout d'une certaine durée de séjour dans l'estomac et libération "pH dépendant" déclenchée par un changement de pH lors de l'entrée des particules dans le petit intestin et qui débute sans temps de latence. Les microparticules de la présente invention sont des microcapsules contenant au moins un Principe Actif (PA) -à l'exclusion du Périndopril -, de granulométrie comprise entre 200 et 800 microns individuellement recouvertes par une pellicule d'enrobage permettant la libération retardée et contrôlée du PA.

Les systèmes à libération retardée et contrôlée de PA sont particulièrement utiles lorsqu'il est souhaitable, pour des raisons de chronobiologie, que le PA soit "bioabsorbé" à une heure précise de la journée afin d'être en phase avec le cycle circadien. Cette approche est appropriée aux traitements du cancer, de l'hypertension, de l'administration de drogues anti inflammatoires ou de la régulation de la glycémie dans le traitement des diabètes. Il peut être par exemple avantageux que le PA soit bioabsorbé très tôt le matin afin d'assurer une couverture thérapeutique au réveil du patient sans pour autant le contraindre à un réveil prématuré. Pour ce faire, le système galénique ingéré par le patient, par exemple le soir après le repas, doit permettre une libération retardée du PA.

Cependant, la première règle imposée au galéniste est de garantir que le médicament prescrit sera absorbé par le patient. Dans le cas d'une forme à libération retardée, il est donc crucial d'avoir une totale garantie de libération du principe actif à un instant déterminé de façon à obtenir l'effet thérapeutique. Or, force est de constater que les formes à libération retardée ne peuvent assurer de façon certaine la libération du PA dans un délai prescrit. Ce problème devient particulièrement aigu dans le cas où il est vital pour le patient que cette libération ait effectivement lieu, comme par exemple celui du traitement des maladies cardiovasculaires ou du diabète.

En effet, de manière conventionnelle, les formes à libération retardée sont obtenues par revêtement du PA par une couche de polymère entérique, par exemple de copolymère d'acide méthacrylique et d'ester méthylique d'acide méthacrylique : EUDRAGIT®L. Ce type de revêtement entérique est connu pour présenter une perméabilité réduite dans les conditions de pH acide de l'estomac et se dissoudre lorsque le pH remonte à une valeur proche de celle régnant dans le petit intestin, libérant ainsi le PA. Cependant, la variabilité intra et interindividuelle des conditions de pH gastrique et de la durée de la vidange gastrique ne permettent pas d'assurer de façon certaine la libération du PA après une durée déterminée.

Les systèmes à libération retardée purement "temps dépendant" c'est à dire pour lesquels la libération du PA se déclenche au bout d'une durée déterminée de séjour dans le tractus gastro-intestinal ne sont pas non plus satisfaisants. En effet, du fait de la variabilité intra et inter individuelle du temps de résidence gastrique, la libération du PA peut se produire après que celui ci soit passé devant sa fenêtre d'absorption, qui est localisée pour une majorité des PA dans la partie haute du tractus gastro-intestinal. La bio-absorption peut ainsi être très faible, voire nulle.

Dans ce contexte, il serait particulièrement avantageux de disposer d'une forme galénique à libération retardée et contrôlée du PA permettant d'assurer de façon certaine la libération du PA grâce à un double mécanisme de déclenchement de la libération du PA : libération "temps dépendant" déclenchée au bout d'une durée contrôlée dans l'estomac, sans changement de pH, et libération "pH dépendant" déclenchée par une remontée du pH lorsque la forme galénique pénètre dans l'intestin. Ces deux facteurs déclencheurs de la libération de PA mis en série conféreraient au système galénique une grande sécurité d'emploi. La libération du PA serait ainsi garantie après un temps de latence préréglé, même si la variation de pH n'est pas intervenue comme déclencheur, c'est-à-dire même si la forme galénique n'est pas passée de l'estomac dans l'intestin.

Afin de minimiser la variabilité interindividuelle de l'absorption du PA, il est nécessaire d'ajuster le temps de latence précédant la libération du PA dans l'estomac en prenant en considération les conditions physiologiques du tractus gastro-intestinal chez l'homme. D'après les résultats bien connus de Davis et al., J. of Controlled Release, 2, 27-38 (1985), le temps de résidence dans l'estomac d'une préparation est très variable, de l'ordre de 0,5 à 10 heures. Il serait donc particulièrement avantageux de disposer d'une forme galénique libérant le principe actif dans l'estomac après un temps de latence donné, constant et compris dans cet intervalle 0,5-10 heures de façon à ce que, d'un individu à l'autre, ou même d'un jour à l'autre pour le même individu, le temps d'action du médicament soit le même.

En outre, afin d'optimiser la biodisponibilité des PA dont l'absorption est limitée principalement aux parties hautes du tractus gastro-intestinal, il serait avantageux que la libération "pH dépendante" dans l'intestin s'effectue sans temps de latence car sinon, le PA ne sera pas libéré dans sa fenêtre d'absorption et par voie de conséquence, le patient ne sera pas traité.

Un autre intérêt unique d'un tel système serait de permettre d'obtenir, par mélange avec une forme galénique à libération immédiate de PA, ou encore par mélange avec une autre forme galénique à libération retardée et contrôlée de PA, des profils de libération présentant plusieurs vagues de libération de PA (un seul ou plusieurs PA identiques ou différents) ou assurant par un réglage adéquat des différentes fractions, un niveau de concentration plasmatique du PA constant.

Il serait aussi avantageux que la forme à libération retardée et contrôlée soit constituée d'une pluralité de microcapsules de diamètre inférieur à 2000 microns. En effet, pour une telle forme, la dose de PA à administrer se répartit entre un grand nombre de microcapsules (typiquement 10 000 pour une dose de 500 mg) et présente de ce fait les avantages intrinsèques suivants :

- Le temps de séjour des microcapsules dans les parties hautes du tractus gastro-intestinal peut être prolongé, ce qui assure un accroissement de la durée de passage du PA devant les fenêtres d'absorption et maximise ainsi la biodisponibilité du PA.
- La mise en oeuvre d'un mélange de microcapsules de profils de libération retardée et contrôlée différents, permet de réaliser des profils de libération présentant plusieurs vagues de libération ou assurant par un réglage adéquat des différentes fractions, un niveau de concentration plasmatique du PA constant.
- La sensibilité à la variabilité de la vidange gastrique est moindre, car la vidange, qui s'effectue ici sur un grand nombre de particules est statistiquement plus reproductible.
- On évite la mise en contact des tissus avec une dose élevée en PA : " dose dumping ". Chaque microcapsule ne contient en effet qu'une dose très réduite en PA. On s'affranchit ainsi du risque de détérioration des tissus par surconcentration locale de PA agressif.
- Il est possible de combiner plusieurs formes galéniques (libération immédiate et/ou retardée et/ou prolongée) comportant un ou plusieurs principes actifs, dans ces systèmes "multimicrocapsulaires".
- Il est possible de présenter ces microcapsules sous forme de sachet, gélule ou comprimé. Dans les cas où la dose de PA est élevée (500 mg ou plus) les formes monolithiques sont de trop grandes dimensions pour être facilement avalées. Il est alors particulièrement intéressant de disposer d'une forme microparticulaire qui assure la libération retardée et contrôlée du PA que l'homme de l'art peut mettre en forme de comprimés délitables ou de sachets.

Enfin, il serait également souhaitable que la pellicule d'enrobage autour des microcapsules soit de faible épaisseur. En effet, un enrobage de forte épaisseur aurait plusieurs conséquences négatives :

   (a) la fraction massique en excipient dans la forme galénique serait trop élevée, d'où une masse de médicament trop importante pour être avalée aisément et donc, in fine, des problèmes d'observance qui mettent en péril le succès du traitement ;
   (b) le temps de fabrication des microcapsules serait très long.

En définitive, il serait donc particulièrement intéressant de disposer d'une forme galénique orale microparticulaire à libération retardée et contrôlée de PA, ayant simultanément les propriétés suivantes :

- la libération du PA peut se déclencher de deux façons :
- par libération dépendante du temps également dénommée "temps dépendant" lorsque la durée de séjour des particules dans l'estomac excède une durée de 5 heures ;
- par libération dépendante d'une variation de pH, également dénommée "pH dépendant", qui débute sans temps de latence lorsque le système pénètre dans l'intestin et que le pH s'accroît. Ces deux facteurs déclencheurs de la libération de PA mis en série garantissent la libération du PA après un temps de latence préréglé, même si la variation de pH n'est pas intervenue comme déclencheur ;
- elle est constituée d'une pluralité de microcapsules de PA enrobé, de petite taille ;
- la fraction massique en excipients d'enrobage est limitée.

La libération retardée ou contrôlée de PA a fait l'objet de nombreux travaux.

Ainsi, la demande EP 0 383 967 A1 divulgue une préparation de diclofénac sodique.

La demande PCT WO-A-96/11675 décrit des microcapsules pour l'administration per os de principes actifs médicamenteux et/ou nutritionnels (PA), dont la taille est inférieure ou égale à 1000 $\mu$m. Ces microcapsules sont constituées par des particules qui sont enrobées par un matériau d'enrobage constitué par un mélange d'un dérivé polymérique filmogène (éthylcellulose), d'un agent plastifiant hydrophobe (huile de ricin), d'un agent tensioactif et/ou lubrifiant (stéarate de magnésium) et d'un polymère azoté (PolyVinylPyrrolidone : PVP). Ces microcapsules sont également caractérisées par leur aptitude à séjourner longtemps (au moins 5 heures) dans l'intestin grêle et à permettre, lors de ce séjour, l'absorption du PA sur une période supérieure au temps de transit naturel dans l'intestin grêle.
Les microcapsules selon cette demande n'apportent pas de solution au problème particulier de la libération retardée et contrôlée de PA, avec un déclenchement "temps dépendant" et "pH dépendant" du PA.

La demande FR-A-00 14876 décrit un médicament de traitement des diabètes de type II, comprenant plusieurs milliers de microcapsules d'anti-hyperglycémiques (metformine) constituées chacune par un coeur comportant au moins un anti-hyperglycémique et par une pellicule d'enrobage (e.g acide stéarique et éthylcellulose) appliquée sur le coeur et permettant la libération prolongée in vivo de l'anti-hyperglycémique. Ces microcapsules ont une granulométrie comprise entre 50 et 1000 $\mu$m.

Cette demande FR-A-00 14876 n'indique pas comment obtenir la libération retardée et contrôlée de PA, avec un déclenchement "temps dépendant" et "pH dépendant" du PA.

La demande de brevet européen EP-A-0 609 961 divulgue des granulés oraux de morphine, permettant la libération contrôlée du PA s'accélérant par la remontée du pH.

Ces granulés comportent :

- un coeur en sucre (Ø = 100 à 1700 $\mu$m),
- enrobé d'une couche d'actif avec un liant (PVP ou HydroxyPropyl-MéthylCellulose : HPMC),
- et une enveloppe extérieure à base :

  ♦ d'un polymère insoluble indépendamment du pH (éthylcellulose ou copolymère d'ester méthacrylique et de méthacrylate d'ammonium: EUDRAGIT® RS ou RL),
  ♦ d'un polymère entérique insoluble à pH acide (copolymère d'acide méthacrylique et d'ester méthylique d'acide méthacrylique: EUDRAGIT® L),
  ♦ d'un composant partiellement soluble à pH acide (polyéthylène glycol, PVP, HPMC, Alcool PolyVinylique: APV),
  ♦ éventuellement un plastifiant (diéthylphtalate),
  ♦ et éventuellement une charge (talc).

Les fractions massiques en PA sont par exemple : 41 %, 38 %, 29 %, et les fractions massiques en enveloppe extérieure e.g. : 14,1 %, 21,5 %, et 12,3 % (poids).

La libération du PA est présente à tout pH et s'amplifie lorsque le pH passe de pH 1,2 à pH 7,5. Il s'agit donc d'une forme à libération prolongée et non retardée.

L'article de H. YOSHINO intitulé "Design and evaluation of time-controlled release systems for site-specific oral drug delivery to the GI tract" paru dans current status on targeted drug delivery to the GI tract, Capsugel library, Symp. Ser., Short Hills 22/04, London 6/05, Tokyo 14/05, p.185-190, (1993), décrit des systèmes galéniques multiparticulaires oraux à libération retardée et contrôlée, induite par un acide organique et par le temps de séjour dans le TGI. Ces systèmes sont constitués de microcapsules de 1000 $\mu$m comportant un coeur neutre en sucre enrobé d'une couche d'actif mélangé avec un acide organique (acide succinique) et une couche externe en copolymère d'ester méthacrylique et de méthacrylate d'ammonium (EUDRAGIT® RS). L'acide organique est décrit comme permettant une libération rapide du PA après la phase de latence ; Cet acide organique est transporté par l'eau ayant pénétré dans les microcapsules dans la couche externe entérique. Il concourt alors à la modification de la perméabilité de l'enrobage, pour permettre la diffusion rapide du PA hors des microcapsules. La présence de cet acide, en contact intime avec le PA peut être préjudiciable à ce dernier.

Le brevet US-B-6,033,687 décrit une formulation constituée par un mélange de deux types de granulés (Ø = 1,4 mm) à base de diltiazem : des granulés à temps de latence court et des granulés à temps de latence long. Les profils de libération sont mesurés à pH 1. Ces granulés comprennent :

► un coeur neutre en sucre (Ø= 0,5-1,5 mm),
► une couche de diltiazem associé à un liant (hydroxypropylcellulose, carboxyméthylcellulose, éthylcellulose, polyvinylpyrrolidone, alginate, EUDRAGIT),
► une couche externe unique à base de lubrifiant (talc), de deux copolymères d'ester méthacrylique et de méthacrylate d'ammonium (EUDRAGIT® RS et EUDRAGIT® RL ; d'un tensioactif (laurylsulfate de sodium) et d'un plastifiant (triéthylcitrate).

Dans les granulés à temps de latence court, la fraction massique de l'enrobage représente 12,3 % contre 30,3 % dans les granulés à temps de latence long. Cette technique ne permet cependant pas d'obtenir des temps de latence longs pour des taux de pelliculage inférieurs à 30 %. Par ailleurs, compte tenu de la variabilité intra et inter individuelle du temps de résidence gastrique, ce système à libération retardée "temps dépendant" peut libérer le PA après que celui-ci soit passé devant sa fenêtre d'absorption. Il en résulte une perte importante de biodisponibilité.

Le brevet EP-B-0 263 083 décrit une composition de revêtement de microcapsules permettant d'obtenir un profil de libération de PA d'ordre zéro et reproductible. Cette composition de revêtement est composée d'un mélange :

○ d'un polymère durcisseur assurant la tenue mécanique du revêtement et pouvant être e.g. : éthylcellulose ou copolymère(s) de l'acide méthacrylique (EUDRAGIT® E, L, S ou RS),
○ d'un composé lipophile, e.g. : acide stéarique ou paraffine,
○ et de talc.

Cette composition de revêtement est présente dans les microcapsules à raison de 15 à 35 % en poids, par exemple. Les ratios polymère durcisseur / composé lipophile sont par exemple de 44 et 42 % respectivement dans les exemples 4 et 5.

Les profils obtenus sont des profils sans temps de latence de durée variable. Il n'est ni enseigné, ni mentionné comment obtenir un profil à libération retardée et contrôlée déclenchée au terme du temps de latence et/ou par une variation du pH.

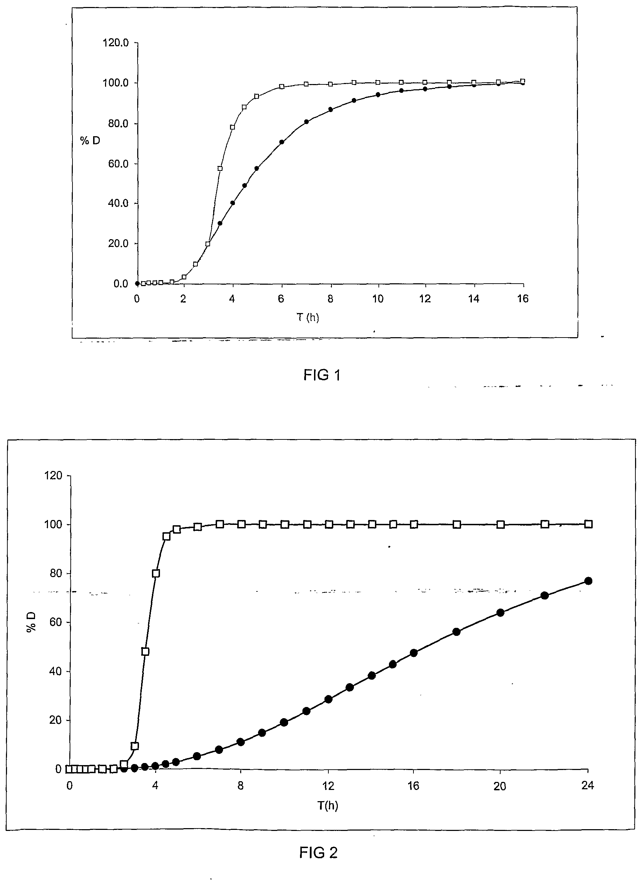

La demande WO-A-01/58424 A1 divulgue des microcapsules "flottantes" enrobées d'un revêtement entérique par exemple à base d'EUDRAGIT® L, de stéarate de magnésium, de talc et d'un plastifiant tel que le dibutylsébaçate. Ce revêtement peut être enveloppé dans une pellicule "bioadhésive" à base de chitosan. Comme tout revêtement entérique, le revêtement entérique selon le document WO-A-01/58424, vise une libération "pH dépendant" et non pas la conjonction d'une libération "temps dépendant" et d'une libération "pH dépendant". Par ailleurs, les figures 1 à 3 de cette demande montrent que le simple objectif de libération "pH dépendant" est très imparfaitement atteint puisque jusqu'à 20 % du PA sont libérés en deux heures seulement à pH acide constant. Les particules décrites dans cette demande flottant dans l'estomac, leur temps de séjour gastrique est décrit comme accru, si bien que l'on peut même craindre l'absence de toute libération "pH déclenchée". Finalement, la libération s'effectuerait de manière incontrôlée par les fuites parasites de PA dans l'estomac.

La demande de brevet européen EP-A-1101490 concerne une préparation pharmaceutique apte à libérer un principe actif dans le gros intestin et plus particulièrement le colon. Cette préparation peut être constituée de comprimés ou de granules comprenant un coeur et un enrobage.

Le problème technique à la base de cette invention est de proposer une forme pharmaceutique apte à permettre la libération d'une substance médicinale dans un site ciblé de la partie inférieure du petit intestin, du colon ascendant, du colon transversal, de la partie inférieure du gros intestin. Compte tenu du fait que le temps moyen de séjour dans l'estomac est de 5 heures et que 2 heures supplémentaires sont, en moyenne, nécessaires pour atteindre la partie inférieure du petit intestin, la préparation selon l'EP-A-1 101 490 est conçue pour que la substance médicinale ne se libère pas pendant 5 heures dans les conditions acides simulant l'estomac et seulement après un temps de latence de 2 heures au moins dans un fluide simulant les conditions de pH de l'intestin (cf. notamment revendication 7 EP-A-1 101 490).

Il apparaît donc que ce système ciblant les substances médicinales absorbées dans les parties inférieures de l'intestin (colon), n'est pas adapté aux substances médicinales absorbées principalement dans les parties hautes du tractus gastro-intestinal. En outre, le système selon la demande de brevet européen EP-A-1 101 490, ne prévoit pas la libération du PA selon un double mécanisme de déclenchement de la libération :

- libération dans l'estomac après en temps de latence donné, constant et compris dans l'intervalle 0,5-10 heures (mécanisme "temps dépendant")

- et libération sans temps de latence après entrée dans l'intestin (mécanisme "pH dépendant").

Enfin, le problème de la variabilité inter ou intra-individuelle du temps de résidence gastrique n'est pas résolu par la préparation selon l'EP-A-1 101 490.

Ainsi l'art antérieur ne comprend pas de système galénique permettant de retarder et de garantir de façon certaine la libération des PA préférentiellement absorbés dans les parties hautes du tractus gastro-intestinal, par un double mécanisme de libération :

- libération "temps dépendant" après un temps de latence dans l'estomac ayant pour caractéristique d'être donné, constant et compris dans l'intervalle 0,5-10 heures

- et libération "pH dépendant" sans temps de latence.

Dans un tel état de la technique, l'un des objectifs essentiels de la présente invention est de fournir un nouveau système galénique multimicroparticulaire pour l'administration orale de principes actifs essentiellement absorbés dans les parties hautes du tractus gastro-intestinal, ce système étant du type à libération retardée et contrôlée assurant la libération du PA de façon certaine et donc garantissant l'efficacité thérapeutique dudit système, grâce à un double mécanisme de libération "temps dépendant" et "pH dépendant". Ces deux facteurs déclencheurs de la libération de PA mis en série garantissent la libération du PA après un temps de latence préréglé, même si la variation de pH n'est pas intervenue comme déclencheur.

Un objectif essentiel de la présente invention est de proposer une forme galénique formée d'une pluralité de microcapsules permettant de se soustraire à la variabilité inter et intra individuelle de la durée de la vidange gastrique, en libérant le PA à pH 1,4 selon un profil de libération retardée présentant un temps de latence de durée donnée ajustable et comprise entre 0,5 et 10 heures, suivi d'une phase de libération débutant sans temps de latence.

Un objectif essentiel de la présente invention est de proposer une forme galénique formée d'une pluralité de microcapsules permettant d'une part de libérer le PA selon un profil de libération retardée à pH 1,4 avec un temps de latence donné, constant et compris entre 0,5-10 heures et selon un temps de demi libération $t_{1/2}$ compris entre 0,25 et 35 heures, et d'autre part, de libérer le PA lorsque le pH passe de 1,4 à 6,8, et ce sans temps de latence et avec un $t_{1/2}$ compris entre 0,25 et 20 heures.

Un objectif essentiel de la présente invention est contrôlé lorsque le pH passe de 1,4 à 6,8.

Un objectif de la présente invention est de proposer une forme galénique constituée d'un grand nombre, par exemple de l'ordre de plusieurs milliers, de microcapsules, cette multiplicité assurant statistiquement une bonne reproductibilité de la cinétique de transit du PA dans tout le tractus gastro-intestinal, de sorte qu'il en résulte un meilleur contrôle de la biodisponibilité et donc une meilleure efficacité.

Un objectif essentiel de la présente invention est de proposer une forme galénique formée d'une pluralité de microcapsules enrobées évitant l'emploi de fortes quantités d'enrobant, la fraction massique d'enrobant étant comparable à celle des formes monolithiques.

Un objectif essentiel de la présente invention est de proposer une forme pharmaceutique formée d'une pluralité de microcapsules enrobées permettant de présenter le PA sous une forme facile à avaler : sachet ou comprimé délitable.

Un objectif essentiel de la présente invention est de proposer une forme pharmaceutique formée d'une pluralité de microcapsules enrobées permettant de mélanger plusieurs principes actifs différents.

Un autre objectif de la présente invention est de proposer une forme pharmaceutique formée d'une pluralité de microcapsules enrobées contenant chacune un coeur neutre.

S'étant fixés les objectifs ci-dessus, parmi d'autres, les inventeurs ont eu le mérite de mettre au point, pour assurer une libération certaine des PA principalement absorbés dans les parties hautes du tractus gastro-intestinal et une bonne bioabsorption des principes actifs pharmaceutiques, un système galénique multimicrocapsulaire :

 ◦ garantissant l'absorption du PA dans sa fenêtre d'absorption, laquelle est limitée principalement aux parties hautes du tractus gastro-intestinal;
 ◦ assurant ainsi une efficacité thérapeutique certaine de ce système ou de cette forme galénique ;
 ◦ et ayant pour caractéristique essentielle un double déclenchement de la libération de PA.

Cela représente un progrès majeur par rapport aux systèmes à libération contrôlée de PA connus jusqu'alors, dans lesquels la libération du PA est déclenchée par un seul facteur : le temps de séjour dans le tractus gastro-intestinal pour certains systèmes, une variation de pH pour d'autres systèmes.

Ainsi, l'invention qui satisfait aux objectifs exposés ci-dessus, parmi d'autres, se rapporte à une forme galénique orale microparticulaire à libération retardée et contrôlée d'au moins un PA -à l'exclusion du périndopril-, ce PA ayant une fenêtre d'absorption in vivo essentiellement limitée aux parties hautes du tractus gastro-intestinal,

ladite forme étant conçue afin de garantir son efficacité thérapeutique en garantissant son absorption in vivo et étant caractérisée :

- en ce que la libération du PA, est régie par deux mécanismes distincts de déclenchement, l'un étant basé sur une variation de pH et l'autre permettant la libération du PA, au bout d'un temps prédéterminé de résidence dans l'estomac,

- et en ce que son comportement de dissolution in vitro (réalisé selon les indications de la pharmacopée européenne 3ème édition intitulée : "Essai de dissolution des formes solides" : dissolutest de type II effectué en conditions SINK maintenu à 37 °C et agité à 100 tours/min), est tel que :

- à pH constant 1,4, le profil de dissolution comporte une phase de latence de durée inférieure ou égale à 5 heures, de préférence comprise entre 1 et 5 heures ;
- le passage, pendant la phase de latence, de pH 1,4 à pH 6,8, conduit à une phase de libération débutant sans temps de latence.

L'invention se rapporte à une forme galénique orale microparticulaire constituée par une pluralité de microcapsules, contenant au moins un Principe Actif (PA) principalement absorbé dans les parties hautes du tractus gastro-intestinal - à l'exclusion du périndopril -, ces microcapsules étant du type de celles :

 ♦ constituées par des particules de PA recouvertes chacune d'au moins une pellicule d'enrobage, cette pellicule d'enrobage étant constituée d'un matériau composite :

  ◦ comprenant :

  • au moins un polymère hydrophile A porteur de groupements ionisés à pH neutre,
  • au moins un composé hydrophobe B ;

  ◦ et représentant une fraction massique (% poids par rapport à la masse totale des microcapsules) $\leq 40$ ;

 ♦ de diamètre inférieur à 2000 microns et de préférence compris entre 200 et 800 microns et, plus préférentiellement encore, entre 200 et 600 microns ;

caractérisées en ce que leur pellicule d'enrobage est constituée par un composite à base de A et de B, dans lequel :

 → le ratio pondéral B/A, est compris entre 0,2 et 1,5, de préférence entre 0,5 et 1,
 → le composé B hydrophobe est sélectionné parmi les produits cristallisés à l'état solide et ayant une température de fusion $T_{fB} \geq 40°C$, de préférence $T_{fB} \geq 50°C$, et plus préférentiellement encore $40°C \leq T_{fB} \leq 90°C$.

Un premier objet de l'invention est ainsi une forme galénique orale microparticulaire à libération retardée et contrôlée d'au moins un principe actif -à l'exclusion du périndopril-, ce principe actif ayant une fenêtre d'absorption in vivo essentiellement limitée aux parties hautes du tractus gastro-intestinal,

cette forme galénique orale comprenant des microcapsules « réservoir » contenant au moins un principe actif -à l'exclusion du périndopril-, ces microcapsules étant du type de celles constituées par des particules de principe actif recouvertes chacune d'au moins une pellicule d'enrobage, et caractérisées

➢ en ce que cette pellicule d'enrobage est constituée d'un matériau composite :

◦ comprenant :

au moins un polymère hydrophile A porteur de groupements ionisés à pH neutre, et au moins un composé hydrophobe B sélectionné parmi le groupe constitué par :

- les cires végétales prises à elles seules ou en mélanges entre elles ;
- les huiles végétales hydrogénées prises à elles seules ou en mélange entre elles ; de préférence choisies dans le groupe comprenant : l'huile de coton hydrogénée, l'huile de soja hydrogénée, l'huile de palme hydrogénée et de tous mélanges entre elles ;
- les diesters du glycérol et d'au moins un acide gras, de préférence l'acide béhénique, pris à eux seuls ou en mélange entre eux ;
- les triesters du glycérol et d'au moins un acide gras, de préférence l'acide béhénique, pris à eux seuls ou en mélange entre eux

◦ et représentant une fraction massique (% poids par rapport à la masse totale des microcapsules) $\leq 40$ ;

➢ en ce que lesdites microcapsules ont un diamètre compris entre 200 et 800 microns, de préférence entre 200 et 600 microns ;
➢ en ce que le ratio pondéral B/A est compris entre 0,5 et 1,5 ;
➢ en ce que la libération du principe actif desdites microcapsules est régie par deux mécanismes distincts de déclenchement, l'un étant basé sur une variation de pH et l'autre permettant la libération du principe actif, au bout d'un temps prédéterminé de résidence dans l'estomac, avec un comportement de dissolution in vitro, réalisée selon les indications de la pharmacopée européenne 3e édition intitulées "Essai de la dissolution des formes orales solides" (dissolutest de type II effectué en conditions SINK maintenu à 37 °C et agité à 100 tours/min), tel que :

- à pH constant 1,4, le profil de dissolution comporte une phase de latence de durée inférieure ou égale à 5 heures, de préférence comprise entre 1 et 5 heures ; et une phase de libération contrôlée suivant la phase de latence telle que le temps de libération de 50 % du principe actif ($t_{1/2}$) est compris entre 0,25 et 35 heures, de préférence entre 0,5 et 20 heures ;
- le passage, pendant la phase de latence, de pH 1,4 à pH 6,8, conduit à une phase de libération débutant sans temps de latence.

Suivant une caractéristique préférée de l'invention, le polymère hydrophile A est choisi parmi :

- les copolymères d'acide (méth)acrylique et d'ester alkyle (e.g méthyle) d'acide (méth)acrylique (EUDRAGIT ® S ou L) et leurs mélanges
- les dérivés de la cellulose, de préférence : cellulose acétate phtalate, hydroxypropylméthylcellulose phtalate, hydroxypropylméthylcellulose acétate succinate ;
- et leurs mélanges.

Les polymères A préférés sont des copolymères d'acide (méth)acrylique et d'esters alkyle (e.g. méthyle) d'acide (méth)acrylique. Ces copolymères, qui sont par exemple du type de ceux commercialisés par la société ROHM PHARMA POLYMERS sous les marques déposées EUDRAGIT® des séries L et S (comme par exemple les EUDRAGIT® L100, S100, L30D-55 et L100-55), sont des (co)polymères, entériques, anioniques et solubles en milieu aqueux à des pH supérieurs à ceux rencontrés dans l'estomac.

Suivant une caractéristique de l'invention, le composé B est choisi parmi le groupe de produits suivants :

- cires végétales prises à elles seules ou en mélanges entre-elles, telles que celles commercialisées sous les marques DYNASAN® P60; DYNASAN® 116, entre autres;
- huiles végétales hydrogénées prises à elles seules ou en mélange entre-elles ; de préférence choisies dans le groupe comprenant : l'huile de coton hydrogénée, l'huile de soja hydrogénée, l'huile de palme hydrogénée et leurs mélanges;
- di ou tri esters du glycérol et d'au moins un acide gras, de préférence l'acide béhénique, pris à eux seuls ou en mélange entre eux ;
- et leurs mélanges.

Le mécanisme de déclenchement de la libération du PA sans variation de pH, au bout d'un temps prédéterminé de résidence dans l'estomac, résulte notamment du contrôle de la vitesse d'hydratation des microcapsules et/ou de la dissolution d'un ou plusieurs composants des microcapsules. Par exemple, et sans vouloir être limitatif, l'hydratation de la microcapsule peut être contrôlée :

○ par la présence, dans les microcapsules, de produits hydrophiles qui permettent d'ajuster la pression osmotique ou de provoquer un gonflement des microcapsules,
○ ou par le réglage de la perméabilité à l'eau de la pellicule d'enrobage;
○ ou par la création d'une microporosité dans la pellicule d'enrobage,
○ ou même par l'hydratation ou la dissolution d'un composé de la pellicule d'enrobage.

L'un des avantages déterminants du système galénique multimicrocapsulaire, à libération retardée et contrôlée de PA, selon l'invention, est de faire intervenir in vivo deux facteurs déclencheurs de la libération du PA dans le tractus gastro-intestinal, à savoir :

- la durée de séjour dans l'estomac : libération "temps déclenchée",
- la variation de pH : libération "pH déclenchée".

Ces deux facteurs déclencheurs de la libération de PA sont en série, de sorte qu'ils confèrent au système galénique une grande sécurité d'emploi. La libération du PA est ainsi garantie après un temps de latence préréglé, même si la variation de pH n'est pas intervenue comme déclencheur. Les problèmes de variabilité interindividuelle sont ainsi surmontés. L'efficacité thérapeutique du médicament comprenant un tel système galénique est assurée, en respectant une chronobiologie prédéterminée et adaptée à la performance thérapeutique visée.

En outre, pour les PA considérés dans la présente invention dont la fenêtre d'absorption est limitée aux parties hautes du tractus gastro-intestinal, il est particulièrement avantageux que la forme à libération retardée puis contrôlée soit une pluralité de microcapsules. En effet, pour une telle forme, la dose de PA à administrer se répartit entre un grand nombre de microcapsules (typiquement 10 000 pour une dose de 500 mg) et présente de ce fait les avantages intrinsèques suivants :

• Le temps de séjour des microcapsules dans les parties hautes du tractus gastro-intestinal peut être prolongé, ce qui assure un accroissement de la durée de passage du PA devant les fenêtres d'absorption et maximise ainsi la biodisponibilité du PA.

• La mise en oeuvre d'un mélange de microcapsules de profils de libération retardée et contrôlée différents permet de réaliser des profils de libération présentant plusieurs vagues de libération ou assurant par un réglage adéquat des différentes fractions, un niveau de concentration plasmatique du PA constant.

• La variabilité de la vidange gastrique est moindre, car la vidange qui s'effectue ici sur un grand nombre de particules est statistiquement plus reproductible.

• On évite la mise en contact des tissus avec une dose élevée en PA : "dose dumping". Chaque microcapsule ne contient en effet qu'une dose très réduite en PA. On s'affranchit ainsi du risque de détérioration des tissus par surconcentration locale de PA agressif.

• Il est possible de présenter ces microcapsules sous forme de sachet, gélule ou comprimé. Dans les cas où la dose de PA est élevée (500 mg ou plus) les formes monolithiques sont de trop grandes dimensions pour être facilement avalées. Il est alors particulièrement intéressant de disposer d'une forme microparticulaire qui assure la libération retardée et contrôlée du PA que l'homme de l'art peut mettre en forme de comprimés délitables ou de sachets.

Le système galénique multimicrocapsulaire selon l'invention permet d'assurer de manière sûre une libération retardée et contrôlée du PA dans le TGI, grâce à deux déclencheurs et de se soustraire ainsi à la variabilité inter et intra individuelle des conditions de la vidange gastrique, tout en étant viable économiquement et facile à ingérer (observance optimisée).

Selon une caractéristique particulièrement avantageuse, à pH constant 1,4, la phase de libération contrôlée suivant la phase de latence est telle que le temps de libération de 50 % poids de PA ($t_{1/2}$) se définit comme suit (en heures) : de préférence

$$0,25 \quad \leq t_{1/2} \leq 35$$

$$0,5 \quad \leq t_{1/2} \leq 20.$$

En pratique, la phase de libération du profil de libération in vitro du PA à pH 1,4 constant, possède un temps de demi-libération qui est ajustable.

Selon un mode de réalisation préféré, la phase de libération suivant le passage de pH 1,4 à pH 6,8, qui s'effectue sans temps de latence, est telle que le temps de libération de 50 % du PA ($t_{1/2}$) se définit comme suit (en heures) : de préférence

$$0,25 \quad \leq t_{1/2} \leq 20$$

$$0,5 \quad \leq t_{1/2} \leq 15.$$

De préférence, les microcapsules selon l'invention comprennent une seule pellicule d'enrobage composite AB. Cela simplifie leur préparation et limite le taux d'enrobage.

De préférence, le PA est déposé sur un coeur neutre de diamètre compris entre 200 et 800 microns et de préférence compris entre 200 et 600 microns.

Sans que cela ne soit limitatif, le coeur neutre hydrophile peut contenir du sucrose et/ou du dextrose et/ou du lactose, ou bien encore être constitué par une microsphère de cellulose.

Avantageusement, l'enrobage des microcapsules peut comprendre outre les constituants essentiels A et B, d'autres ingrédients classiques et connus de l'homme du métier, tels que notamment :

- des colorants ;
- des plastifiants comme par exemple le dibutylsébaçate ;
- des composés hydrophiles comme par exemple la cellulose et ses dérivés ou la polyvinylpyrrolidone et ses dérivés ;
- et leurs mélanges.

Avantageusement, le PA est déposé par les techniques connues de l'homme de l'art, par exemple la technique de spray coating en lit d'air fluidisé, sur des coeurs neutres de diamètre compris entre 200 et 800 microns et de préférence 200 et 600 microns.

Sur le plan quantitatif, la monocouche d'enrobant représente au plus 40 %, de préférence au plus 30 % en poids des microcapsules. Un tel taux limité d'enrobage permet de réaliser des unités galéniques contenant chacune une haute dose de principe actif, sans dépasser une taille rédhibitoire au regard de la déglutition. L'observance et donc le succès du traitement ne peuvent que s'en trouver améliorés.

Qualitativement parlant, le PA des microcapsules selon l'invention est absorbable essentiellement dans les parties hautes du tractus gastro-intestinal et il est avantageusement choisi parmi l'une des familles de substances actives suivantes : antiulcéreux, antidiabétiques, anticoagulants, antithrombiques, hypolipémiants, antiarythmiques, vasodilatateurs, antiangineux, antihypertenseurs, vasoprotecteurs, promoteurs de fécondité, inducteurs et inhibiteurs du travail utérin, contraceptifs, antibiotiques, antifongiques, antiviraux, anticancéreux, anti-inflammatoires, analgésiques, anti-épileptiques, antiparkinsoniens, neuroleptiques, hypnotiques, anxiolytiques, psychostimulants, antimigraineux, antidépresseurs, antitussifs, antihistaminiques ou antiallergiques.

On peut également se référer à la liste de principes actifs donnés dans la demande EP-A-0 609 961 aux pages 4 à 8.

De manière préférée, le PA est sélectionné parmi les composés suivants : metformine, acide acétylsalicylique, amoxicilline, pentoxifylline, prazosine, acyclovir, nifedipine, diltiazem, naproxen, ibuprofen, flurbiprofen, ketoprofen, fenoprofen, indométhacine, diclofenac, fentiazac, oestradiol valérate, métoprolol, sulpiride, captopril, cimetidine, zidovudine, nicardipine, terfenadine, atenolol, salbutamol, carbamazepine, ranitidine, énalapril, simvastatine, fluoxétine, alprazolam, famotidine, ganciclovir, famciclovir, spironolactone, 5-asa, quinidine, morphine, pentazocine, paracétamol, oméprazole, métoclopramide et leurs mélanges.

La forme galénique orale microparticulaire selon l'invention peut être un comprimé, avantageusement orodispersible, une poudre ou une gélule.

Les microcapsules décrites ci-dessus peuvent être utilisées pour la fabrication de nouvelles préparations pharmaceutiques ou diététiques de divers PA, ayant des performances thérapeutiques ou diététiques optimisées et se présentant de préférence sous forme de comprimés avantageusement délitables et plus préférablement encore orodispersibles, de poudres ou de gélules.

Ces microcapsules sont d'autant plus intéressantes qu'elles sont en outre parfaitement tolérées par l'organisme, notamment au niveau gastrique et par ailleurs peuvent être obtenues de façon aisée et économique.

La présente invention concerne, en outre, ces nouvelles préparations pharmaceutiques ou diététiques en tant que telles, originales dans leur structure, leur présentation et leur composition. De telles préparations pharmaceutiques ou diététiques sont administrées per os, de préférence par doses journalières uniques.

Il est à noter qu'il peut être intéressant de mélanger dans une même gélule, un même comprimé ou une même poudre, au moins deux types de microcapsules à cinétiques de libération différentes mais comprises dans le cadre caractéristique de l'invention.

On peut également mélanger les microcapsules selon l'invention avec une certaine quantité de PA immédiatement disponible dans l'organisme.

Il est également envisageable d'associer des microcapsules contenant des PA différents.

En outre, un autre objet de l'invention est un système galénique (pharmaceutique ou diététique), de préférence sous forme de comprimé, avantageusement délitable et plus préférablement encore orodispersibles, de poudre ou de gélule, caractérisé en ce qu'il comprend des microcapsules, telles que décrites supra.

Par ailleurs, l'invention vise l'utilisation des microparticules telles que définies ci-dessus, pour la préparation de formes galéniques orales microparticulaires, pharmaceutiques ou diététiques, de préférence sous forme de comprimés avantageusement orodispersibles, de poudres ou de gélules.

L'invention sera mieux expliquée par les exemples ci-après, donnés uniquement à titre d'illustration et permettant de bien comprendre l'invention et de faire ressortir ses variantes de réalisation et/ou de mise en oeuvre, ainsi que ses différents avantages.

**EXEMPLES**

Description des figures :

- **La figure 1** représente les profils de libération in vitro des microcapsules de l'exemple 1, à pH 1,4 : ⎯●⎯ et à pH 1,4 pendant 3 heures puis à pH 6,8 à partir de T = 3 heures : —☐— , en % en poids (% D) de metformine dissoute en fonction du temps T en heures ;

- **La figure 2** représente les profils de libération in vitro des microcapsules de l'exemple 2, à pH 1,4 : —●— , et à pH 1,4 pendant 2 heures puis à pH 6,8 à partir de 2 heures : —☐—, en % en poids (% D) d'acyclovir en fonction du temps T en heures ;
- **La figure 3** représente les profils de libération in vitro des microcapsules de l'exemple 3, à pH 1,4 : —■— et à pH 6,8 : —☐—, en % en poids (% D) de metformine en fonction du temps T en heures.

EXEMPLES

**Exemple 1 : Préparation de microcapsules conduisant à une libération de metformine, HCl retardée et prolongée à double mécanisme**

75 g de Metformine, HCl (Chemsource) et 75 g de PVP sont dissous dans 1350 g d'isopropanol. La solution est pulvérisée sur 850 g de microsphères neutres (NP Pharm). dans un spray coater Glatt® GPCG3.

93,3 g d'huile de palme hydrogénée (Hüls) -B- et 140 g d'Eudragit® L100 (Röhm) -A-sont dissous à chaud dans de l'isopropanol. B/A = 0,66. La solution est pulvérisée sur 700 g de microparticules préparées précédemment. Les conditions de pelliculage sont : Température d'entrée : 45°C, débit de pulvérisation : 8-12 g/min, pression d'atomisation : 1,5 bar.

Les microcapsules ont été testées dans un dissolutest de type II conforme à la Pharmacopée à 37°C et sous une agitation de 100 tours/min dans les milieux suivants :

    a) HCl à pH 1,4
    b) HCl à pH 1,4 pendant 3 heures puis milieu tampon $KH_2PO_4$ / NaOH à pH 6,8

Les profils de libération sont présentés sur la figure 1.

Ces profils sont caractéristiques d'une libération retardée puis prolongée à double mécanisme : absence de libération pendant 2 heures suivie par une libération prolongée sans changement de pH et suivie enfin par une libération accélérée par le changement de pH.

**Exemple 2 : Préparation de microcapsules conduisant à une libération d'Acyclovir retardée et prolongée à double mécanisme**

75 g d'Acyclovir et 75 g de PolyvinylPyroolidone PLASDONE® K29/32 sont dissous dans 833 g d'isopropanol. La solution est pulvérisée sur 850 g de microsphères neutres (NP Pharm) dans un spray coater Glatt® GPCG3.

93,3 g d'huile de palme hydrogénée (Hüls) -B- et 140 g d'EUDRAGIT® L100 (Röhm) - A- sont dissous à chaud dans de l'isopropanol. B/A = 0,66. La solution est pulvérisée sur 700 g de microparticules préparées précédemment. Les conditions de pelliculage sont : Température d'entrée : 45°C, débit de pulvérisation : 8-12 g/min, pression d'atomisation : 1,5 bar.

Les microcapsules ont été testées dans un dissolutest de type II conforme à la Pharmacopée à 37°C et sous une agitation de 100 tours/min dans les milieux suivants :

    c) HCl à pH 1,4
    d) HCl à pH 1,4 pendant 3 heures puis milieu tampon $KH_2PO_4$ / NaOH à pH 6,8

Les profils de libération sont présentés sur la figure 2.

Le profil de libération de l'Acyclovir obtenu à pH 1,4 est caractéristique d'une libération retardée et prolongée, à double mécanisme de déclenchement de la libération.

**Exemple 3 : Préparation de microcapsules conduisant à une libération de metformine, HCl retardée et prolongée à double mécanisme**

105 g d'huile de palme hydrogénée (Hüls) -B-, 30 g de dibutyl sébaçate et 165 g d'Eudragit® L100 (Röhm) -A- sont dissous à chaud dans de l'isopropanol. B/A = 0,64. La solution est pulvérisée sur 700 g de granulés de Metformine (95,5% Metformine / 4,5% PVP). Les conditions de pelliculage sont : Température d'entrée : 45°C, débit de pulvérisation : 8-12 g/min, pression d'atomisation : 1,5 bar.

Les microcapsules ont été testées dans un dissolutest de type II conforme à la Pharmacopée à 37°C et sous une agitation de 100 tours/min dans les milieux suivants :

    e) HCl à pH 1,4
    f) milieu tampon $KH_2PO_4$ / NaOH à pH 6,8

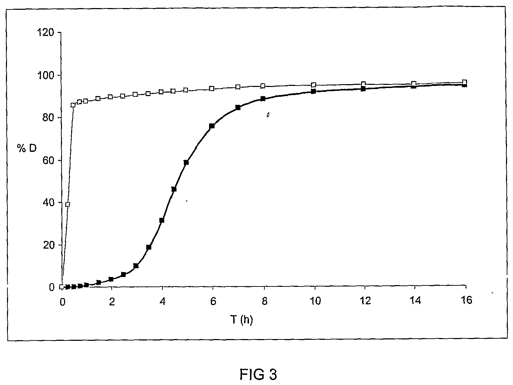

Les profils de libération sont présentés sur la figure 3.

Ces profils sont caractéristiques d'une libération retardée puis prolongée à double mécanisme : absence de libération pendant 2 heures à pH acide et libération rapide à pH neutre.

**Revendications**

1.    Forme galénique orale microparticulaire à libération retardée et contrôlée d'au moins un principe actif -à l'exclusion du périndopril-, ce principe actif ayant une fenêtre d'absorption in vivo essentiellement limitée aux parties hautes du tractus gastro-intestinal, cette forme galénique orale comprenant des microcapsules « réservoir » contenant au moins un principe actif-à l'exclusion du périndopril-, ces microcapsules étant du type de celles constituées par des particules de principe actif recouvertes chacune d'au moins une pellicule d'enrobage, et caractérisées

➢ en ce que cette pellicule d'enrobage est constituée d'un matériau composite :

◦ comprenant :

au moins un polymère hydrophile A porteur de groupements ionisés à pH neutre, et
au moins un composé hydrophobe B sélectionné parmi le groupe constitué par :

- les cires végétales prises à elles seules ou en mélanges entre elles ;
- les huiles végétales hydrogénées prises à elles seules ou en mélange entre elles ; de préférence choisies dans le groupe comprenant : l'huile de coton hydrogénée, l'huile de soja hydrogénée, l'huile de palme hydrogénée et de tous mélanges entre elles;
- les diesters du glycérol et d'au moins un acide gras, de préférence l'acide béhénique, pris à eux seuls ou en mélange entre eux ;
- les triesters du glycérol et d'au moins un acide gras, de préférence l'acide béhénique, pris à eux seuls ou en mélange entre eux

◦ et représentant une fraction massique (% poids par rapport à la masse totale des microcapsules) $\leq 40$ ;

➢ en ce que lesdites microcapsules ont un diamètre compris entre 200 et 800 microns, de préférence entre 200 et 600 microns;
➢ en ce que le ratio pondéral B/A est compris entre 0,5 et 1,5;
➢ en ce que la libération du principe actif desdites microcapsules est régie par deux mécanismes distincts de déclenchement, l'un étant basé sur une variation de pH et l'autre permettant la libération du principe actif, au bout d'un temps prédéterminé de résidence dans l'estomac, avec un comportement de dissolution in vitro, réalisée selon les indications de la pharmacopée européenne 3e édition intitulées "Essai de la dissolution des formes orales solides" (dissolutest de type II effectué en conditions SINK maintenu à 37 °C et agité à 100 tours/min),tel que :

- à pH constant 1,4, le profil de dissolution comporte une phase de latence de durée inférieure ou égale à 5 heures, de préférence comprise entre 1 et 5 heures ; et une phase de libération contrôlée suivant la phase de latence telle que le temps de libération de 50 % du principe actif ($t_{1/2}$) est compris entre 0,25 et 35 heures, de préférence entre 0,5 et 20 heures ;
- le passage, pendant la phase de latence, de pH 1,4 à pH 6,8, conduit à une phase de libération débutant sans temps de latence.

2. Forme galénique selon la revendication 1, **caractérisée en ce que** le ratio pondéral B/A est compris entre 0,5 et 1,0.

3. Forme galénique selon la revendication 1 ou 2, **caractérisée en ce que** le polymère hydrophile A est choisi parmi :

- les copolymères d'acide (méth)acrylique et d'ester alkyle d'acide (méth)acrylique et leurs mélanges;
- les dérivés de la cellulose, de préférence : cellulose acétate phtalate, hydroxypropylméthylcellulose phtalate, hydroxypropylméthylcellulose acétate succinate ;
- et leurs mélanges.

4. Forme galénique selon l'une quelconque des revendications 1 à 3, **caractérisée en ce que** le composé B est choisi parmi le groupe de produits suivants :

- cires végétales prises à elles seules ou en mélanges entre elles ;
- huiles végétales hydrogénées prises à elles seules ou en mélange entre elles ; de préférence choisies dans le groupe comprenant : l'huile de coton hydrogénée, l'huile de soja hydrogénée, l'huile de palme hydrogénée et de tous mélanges entre elles;
- et leurs mélanges.

5. Forme galénique selon l'une quelconque des revendications 1 à 4, **caractérisée en ce que** la phase de libération suivant le passage de pH 1,4 à pH 6,8, qui s'effectue sans temps de latence, est telle que le temps de libération de 50 % du principe actif ($t_{1/2}$) se définit comme suit (en heures) : de préférence

$$0,25 \leq t_{1/2} \leq 20$$

$$0,5 \quad \leq t_{1/2} \leq 15.$$

**6.** Forme galénique selon l'une quelconque des revendications 1 à 5, **caractérisée en ce que** les microcapsules comprennent une seule pellicule d'enrobage composite AB.

**7.** Forme galénique selon l'une quelconque des revendications 1 à 6, **caractérisée en ce que** le principe actif est déposé sur un coeur neutre de diamètre compris entre 200 et 600 microns.

**8.** Forme galénique selon la revendication 7, **caractérisée en ce que** le coeur neutre contient du sucrose et/ou du dextrose et/ou du lactose.

**9.** Forme galénique selon la revendication 7, **caractérisée en ce que** le coeur neutre est une microsphère de cellulose.

**10.** Forme galénique selon l'une quelconque des revendications 1 à 9, **caractérisée en ce que** le principe actif mis en oeuvre appartient à au moins l'une des familles de substances actives suivantes : antiulcéreux, antidiabétiques, anticoagulants, antithrombiques, hypolipémiants, antiarythmiques, vasodilatateurs, antiangineux, antihypertenseurs, vasoprotecteurs, promoteurs de fécondité, inducteurs et inhibiteurs du travail utérin, contraceptifs, antibiotiques, antifongiques, antiviraux, anticancéreux, anti inflammatoires, analgésiques, antiépileptiques, antiparkinsoniens, neuroleptiques, hypnotiques, anxiolytiques, psychostimulants, antimigraineux, antidépresseurs, antitussifs, antihistaminiques ou antiallergiques.

**11.** Forme galénique selon la revendication 10, **caractérisées en ce que** le principe actif est choisi parmi les composés suivants : amoxicilline, metformine, acide acétylsalicylique, pentoxifylline, prazosine, acyclovir, nifedipine, diltiazem, naproxen, ibuprofen, flurbiprofen, ketoprofen, fenoprofen, indométhacine, diclofenac, fentiazac, oestradiol valérate, métoprolol, sulpiride, captopril, cimetidine, zidovudine, nicardipine, terfenadine, atenolol, salbutamol, carbamazepine, ranitidine, énalapril, simvastatine, fluoxétine, alprazolam, famotidine, ganciclovir, famciclovir, spironolactone, 5-asa, quinidine, morphine, pentazocine, paracétamol, oméprazole, métoclopramide et leurs mélanges.

**12.** Forme galénique selon l'une quelconque des revendications 1 à 11, **caractérisée en ce qu'**elle est un comprimé, avantageusement orodispersible, une poudre ou une gélule.

**13.** Utilisation des microcapsules telles que définies dans l'une quelconque des revendications 1 à 11 pour la préparation de formes galéniques orales microparticulaires, pharmaceutiques ou diététiques, de préférence sous forme de comprimés avantageusement orodispersibles, de poudres ou de gélules.

**Patentansprüche**

**1.** Orale mikropartikuläre Dosierungsform für die verzögerte und kontrollierte Freisetzung mindestens eines Wirkstoffs - mit Ausnahme von Perindopril -, wobei der Wirkstoff in vivo ein Absorptionsfenster besitzt, das im Wesentlichen auf den oberen Magen-Darm-Trakt beschränkt ist,
wobei die orale Dosierungsform "Reservoir"-Mikrokapseln umfasst, die mindestens einen Wirkstoff - mit Ausnahme von Perindopril - enthalten, wobei die Mikrokapseln von demjenigen Typ sind, der aus Wirkstoffpartikeln besteht, die jeweils mit mindestens einem Beschichtungsfilm beschichtet sind, und **dadurch gekennzeichnet, dass**

➢ der Beschichtungsfilm aus einem Verbundmaterial besteht:

◦ das Folgendes umfasst:

mindestens ein hydrophiles Polymer A, das bei neutralem pH ionisierte Gruppen trägt, und
mindestens eine hydrophobe Verbindung B, die aus der Gruppe ausgewählt ist, bestehend aus:

- pflanzlichen Wachsen jeweils allein oder in Gemischen miteinander,
- hydrierten pflanzlichen Ölen jeweils allein oder im Gemisch miteinander, die vorzugsweise aus der Gruppe ausgewählt sind, die Folgende umfasst: hydriertes Baumwollöl, hydriertes Sojaöl, hydriertes Palmöl und sämtliche Gemische zwischen diesen,
- Diestern von Glycerin und mindestens einer Fettsäure, vorzugsweise Behensäure, jeweils allein oder im Gemisch miteinander,
- Triestern von Glycerin und mindestens einer Fettsäure, vorzugsweise Behensäure, jeweils allein oder im Gemisch miteinander,

◦ und eine Massenfraktion (Gew.-% in Bezug auf die Gesamtmasse der Mikrokap-

seln) $\leq$ 40 darstellt;

➢ dass die Mikrokapseln einen Durchmesser zwischen 200 und 800 Mikron, vorzugsweise zwischen 200 und 600 Mikron besitzen,
➢ dass das Gewichtsverhältnis B/A zwischen 0,5 und 1,5 beträgt,
➢ dass die Freisetzung des Wirkstoffs aus den Mikrokapseln durch zwei verschiedene Auslösemechanismen reguliert wird, von denen einer auf einer Veränderung des pH basiert und der andere die Freisetzung des Wirkstoffs nach Ablauf einer festgelegten Verweildauer im Magen gestattet, mit einem Löseverhalten in vitro, das gemäß den Angaben im Europäischen Arzneibuch, 3. Auflage, mit dem Titel "Prüfung der Dissolution fester oraler Formen" (Dissolutest Typ II, durchgeführt unter SINK-Bedingungen sowie Halten bei 37°C und unter Rühren bei 100 U/min) durchgeführt wird, derart, dass:

- bei konstantem pH von 1,4 das Lösungsprofil eine Latenzphase mit einer Dauer von weniger als oder gleich 5 Stunden, vorzugsweise zwischen 1 und 5 Stunden, und eine auf die Latenzphase folgende Phase der kontrollierten Freisetzung umfasst, derart, dass die Zeit für die Freisetzung von 50% des Wirkstoffs ($t_{1/2}$) zwischen 0,25 und 35 Stunden, vorzugsweise zwischen 0,5 und 20 Stunden beträgt,
- der Übergang während der Latenzphase von pH 1,4 auf pH 6,8 zu einer Freisetzungsphase führt, die ohne Latenzzeit beginnt.

2. Dosierungsform nach Anspruch 1, **dadurch gekennzeichnet, dass** das Gewichtsverhältnis B/A zwischen 0,5 und 1,0 beträgt.

3. Dosierungsform nach Anspruch 1 oder 2, **dadurch gekennzeichnet, dass** das hydrophile Polymer A aus den Folgenden ausgewählt ist:

- Copolymeren von (Meth)Acrylsäure und (Meth)Acrylsäurealkylester und deren Gemischen,
- Derivaten von Cellulose, vorzugsweise: Celluloseacetatphthalat, Hydroxypropylmethylcellulosephthalat, Hydroxypropylmethylcelluloseacetatsuccinat
- und deren Gemischen.

4. Dosierungsform nach einem der Ansprüche 1 bis 3, **dadurch gekennzeichnet, dass** die Verbindung B aus der Gruppe der folgenden Produkte ausgewählt ist:

- pflanzlichen Wachsen jeweils allein oder in Gemischen miteinander,
- hydrierten pflanzlichen Ölen jeweils allein oder im Gemisch miteinander, die vorzugsweise aus der Gruppe ausgewählt sind, die Folgende umfasst: hydriertes Baumwollöl, hydriertes Sojaöl, hydriertes Palmöl und sämtliche Gemische zwischen diesen,
- und deren Gemischen.

5. Dosierungsform nach einem der Ansprüche 1 bis 4, **dadurch gekennzeichnet, dass** die Freisetzungsphase, die auf den Übergang von pH 1,4 nach pH 6,8 folgt, die ohne Latenzphase erfolgt, derart ist, dass die Zeit für die Freisetzung von 50% des Wirkstoffs ($t_{1/2}$) folgendermaßen definiert ist (in Stunden): vorzugsweise

$$0,25 \leq t_{1/2} \leq 20,$$

$$0,5 \leq t_{1/2} \leq 15.$$

6. Dosierungsform nach einem der Ansprüche 1 bis 5, **dadurch gekennzeichnet, dass** die Mikrokapseln einen einzigen AB-Verbund-Beschichtungsfilm umfassen.

7. Dosierungsform nach einem der Ansprüche 1 bis 6, **dadurch gekennzeichnet, dass** der Wirkstoff auf einen neutralen Kern mit einem Durchmesser zwischen 200 und 600 Mikron aufgebracht ist.

8. Dosierungsform nach Anspruch 7, **dadurch gekennzeichnet, dass** der neutrale Kern Saccharose und/oder Dextrose und/oder Lactose enthält.

9. Dosierungsform nach Anspruch 7, **dadurch gekennzeichnet, dass** der neutrale Kern eine Cellulose-Mikrosphäre ist.

10. Dosierungsform nach einem der Ansprüche 1 bis 9, **dadurch gekennzeichnet, dass** der verwendete Wirkstoff zu mindestens einer der folgenden Wirkstofffamilien gehört: Mitteln gegen Geschwüre, Antidiabetika, Antikoagulantien, Antithrombotika, Lipidsenkern, Antiarrhythmika, Vasodilatatoren, Antianginosa, Antihypertonika, Vasoprotektoren, die Fruchtbarkeit fördernden Mitteln, Weheninduktoren und -inhibitoren, Kontrazeptiva, Antibiotika, Antimykotika, antiviralen Mitteln, Antikrebsmitteln, entzündungshemmenden Mitteln, Analgetika, Antiepileptika, Mitteln gegen Parkinson, Neuroleptika, Hypnotika, Anxiolytika, Psychostimulantien, Mitteln gegen Migräne, Antidepressiva, Mitteln gegen Husten, Antihistaminika oder Antiallergika.

**11.** Dosierungsform nach Anspruch 10, **dadurch gekennzeichnet, dass** der Wirkstoff aus den folgenden Verbindungen ausgewählt ist: Amoxicillin, Metformin, Acetylsalicylsäure, Pentoxifyllin, Prazosin, Acyclovir, Nifedipin, Diltiazem, Naproxen, Ibuprofen, Flurbiprofen, Ketoprofen, Fenoprofen, Indometacin, Diclofenac, Fentiazac, Östradiolvalerat, Metoprolol, Sulpirid, Captopril, Cimetidin, Zidovudin, Nicardipin, Terfenadin, Atenolol, Salbutamol, Carbamazepin, Ranitidin, Enalapril, Simvastatin, Fluoxetin, Alprazolam, Famotidin, Ganciclovir, Famciclovir, Spironolacton, 5-Asa, Chinidin, Morphin, Pentazocin, Paracetamol, Omeprazol, Metoclopramid und deren Gemische.

**12.** Dosierungsform nach einem der Ansprüche 1 bis 11, **dadurch gekennzeichnet, dass** sie eine, vorteilhafterweise in der Mundhöhle dispergierbare, Tablette, ein Pulver oder eine Kapsel ist.

**13.** Verwendung von Mikrokapseln nach einem der Ansprüche 1 bis 11 zur Herstellung von oralen mikropartikulären, pharmazeutischen oder diätetischen Dosierungsformen vorzugsweise in Form von, vorteilhafterweise in der Mundhöhle dispergierbaren, Tabletten, Pulvern oder Kapseln.

**Claims**

**1.** Microparticulate oral dosage form for the delayed and controlled release of at least one active ingredient -excluding perindopril-, said active ingredient having an absorption window in vivo that is essentially limited to the upper parts of the gastrointestinal tract,
said oral dosage form comprising "reservoir" microcapsules containing at least one active ingredient -excluding perindopril-, said microcapsules being of the type of those which consist of active ingredient particles each coated with at least one coating film, and characterised

    ➢ in that this coating film consists in a composite material:

        o comprising:

            at least one hydrophilic polymer A carrying groups that are ionized at neutral pH, and
            at least one hydrophobic compound B selected from the group consisting of:

                - vegetable waxes taken on their own or in mixtures with one another;
                - hydrogenated vegetable oils taken on their own or in a mixture with one another; preferably selected from the group comprising: hydrogenated cottonseed oil, hydrogenated soybean oil, hydrogenated palm oil and any mixtures thereof;
                - diesters of glycerol with at least one fatty acid, preferably behenic acid, taken by themselves or in a mixture with one another;
                - triesters of glycerol with at least one fatty acid, preferably behenic acid, taken by themselves or in a mixture with one another

        o and representing a mass fraction (% by weight relative to the total mass of the microcapsules) of ≤ 40;

    ➢ in that said microcapsules have a diameter comprised between 200 and 800 microns, preferably between 200 and 600 microns;
    ➢ in that the weight ratio B/A is comprised between 0.5 and 1.5;
    ➢ in that the release of the active ingredient from said microcapsules is governed by two distinct triggering mechanisms, one being based on a variation in pH and the other allowing the release of the active ingredient, after a predetermined residence time in the stomach, with a dissolution behavior in vitro determined as indicated in the European Pharmacopeia, 3rd edition, under the title: "Dissolution test for solid oral forms" (type II dissolutest performed under SINK conditions, maintained at 37°C and stirred at 100 rpm), such that:

        - at a constant pH of 1.4, the dissolution profile includes a latency phase with a duration less than or equal to 5 hours, preferably comprised between 1 and 5 hours; and a controlled release phase following the latency phase such that the release time for 50% of the active ingredient ($t_{1/2}$) is comprised between 0.25 and 35 hours, preferably between 0.5 and 20 hours;
        - the change, during the latency phase, from pH 1.4 to pH 6.8 results in a release phase that starts without a latency period.

**2.** Dosage form according to claim 1, **characterised in that** the weight ratio B/A is comprised between 0.5 and 1.0.

**3.** Dosage form according to claim 1 or 2, **characterised in that** the hydrophilic polymer A is selected from:

- copolymers of (meth)acrylic acid and alkyl (meth)acrylate and mixtures thereof;
- cellulose derivatives, preferably: cellulose acetate phthalate, hydroxypropyl methyl cellulose phthalate and hydroxypropyl methyl cellulose acetate succinate;
- and mixtures thereof.

4. Dosage form according to any one of claims 1 to 3, **characterised in that** the compound B is selected from the following group of products:

- vegetable waxes, taken on their own or in mixtures with one another;
- hydrogenated vegetable oils taken on their own or in a mixture with one another; preferably selected from the group comprising: hydrogenated cottonseed oil, hydrogenated soybean oil, hydrogenated palm oil and any mixture thereof;
- and mixtures thereof.

5. Dosage form according to any one of claims 1 to 4, **characterised in that** the release phase following the change from pH 1.4 to pH 6.8, that takes place without a latency period, is such that the release time for 50% of the active ingredient ($t_{1/2}$) is defined as follows (in hours): preferably

$$0.25 \leq t_{1/2} \leq 20$$

$$0.5 \leq t_{1/2} \leq 15$$

6. Dosage form according to any one of claims 1 to 5, **characterised in that** the microcapsules comprise a single AB composite coating film.

7. Dosage form according to any one of claims 1 to 6, **characterised in that** the active ingredient is deposited on a neutral core with a diameter comprised between 200 and 600 microns.

8. Dosage form according to claim 7, **characterised in that** the neutral core contains sucrose and/or dextrose and/or lactose.

9. Dosage form according to claim 7, **characterised in that** the neutral core is a cellulose micro sphere.

10. Dosage form according to any one of claims 1 to 9, **characterised in that** the active ingredient used belongs to at least one of the following families of active substances: antiulcer agents, antidiabetics, anticoagulants, antithrombics, hypolipidemics, antiarrhythmics, vasodilators, antiangina agents, antihypertensives, vasoprotectors, fertility promoters, uterine labour inducers and inhibitors, contraceptives, antibiotics, antifungals, antivirals, anticancer agents, anti-inflammatories, analgesics, antiepileptics, antiparkinsonian agents, neuroleptics, hypnotics, anxiolytics, psychostimulants, antimigraine agents, antidepressants, antitussives, antihistamines and antiallergics.

11. Dosage form according to claim 10, **characterised in that** the active ingredient is selected from the following compounds: amoxicillin, metformin, acetylsalicylic acid, pentoxifyllin, prazosin, acyclovir, nifedipine, diltiazem, naproxen, ibuprofen, flurbiprofen, ketoprofen, fenoprofen, indomethacin, diclofenac, fentiazac, estradiol valerate, metoprolol, sulpiride, captopril, cimetidine, zidovudine, nicardipine, terfenadine, atenolol, salbutamol, carbamazepine, ranitidine, enalapril, simvastatin, fluoxetine, alprazolam, famotidine, ganciclovir, famciclovir, spironolactone, 5-asa, quinidine, morphine, pentazocine, acetaminophen, omeprazole, metoclopramide and mixtures thereof.

12. Dosage form according to any one of claims 1 to 11, **characterised in that** it is a tablet, advantageously an orodispersible tablet, a powder or a gelatin capsule.

13. Use of microcapsules such as defined in any one of claims 1 to 11 for the preparation of microparticulate pharmaceutical or dietetic oral dosage forms, preferably as tablets, advantageously orodispersible tablet, powders or gelatin capsules.

FIG 1

FIG 2

FIG 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0383967 A1
- WO 9611675 A
- FR 0014876 A
- EP 0609961 A
- US 6033687 B

- EP 0263083 B
- WO A0158424 A1
- WO 0158424 A
- EP 1101490 A

**Littérature non-brevet citée dans la description**

- **DAVIS et al.** *J. of Controlled Release,* 1985, vol. 2, 27-38

- **H. YOSHINO.** ''Design and evaluation of time-controlled release systems for site-specific oral drug delivery to the GI tract'' paru dans current status on targeted drug delivery to the GI tract. *Capsugel library, Symp. Ser.,* 1993, 185-190